# EUROPÄISCHE PATENTSCHRIFT

(11) **EP 1 556 012 B1**
(45) Veröffentlichungstag und Bekanntmachung des Hinweises auf die Patenterteilung: **29.08.2007**
(21) Anmeldenummer: 03775176.5
(22) Anmeldetag: 09.10.2003
(51) Int. Cl.: A61K 9/16, A61K 31/513

(54) **HERSTELLUNG VON FESTEN DOSIERUNGSFORMEN UNTER VERWENDUNG EINES VERNETZTEN NICHT-THERMOPLASTISCHEN TR GERS**
METHOD FOR PRODUCING SOLID GALENIC FORMULATIONS USING A CROSSLINKED NON-THERMOPLASTIC CARRIER
PRODUCTION DE FORMES GALENIQUES SOLIDES AU MOYEN D'UN EXCIPIENT NON THERMOPLASTIQUE RETICULE

(30) Priorität: 09.10.2002 DE 10247037
(43) Veröffentlichungstag der Anmeldung: 27.07.2005
(73) Patentinhaber: Abbott GmbH & Co. KG, 65205 Wiesbaden (DE)
(72) Erfinder: ROSENBERG, Jörg, 67158 Ellerstadt (DE); BERNDL, Gunther, 67273 Herxheim (DE); MÄGERLEIN, Markus, 68167 Mannheim (DE)
(74) Vertreter: Thalhammer, Wolfgang
(86) Internationale Anmeldenummer: PCT/EP2003/011205
(87) Internationale Veröffentlichungsnummer: WO 2004/032903

(56) Entgegenhaltungen:
- EP-A- 0 960 620
- WO-A-01/78716
- GB-A- 1 442 951
- US-A1- 2002 012 706

## Beschreibung

Die vorliegende Erfindung betrifft ein Verfahren zur Herstellung von rasch freisetzenden festen Dosierungsformen.

Die Herstellung fester Dosierungsformen durch Schmelzextrusion, d. h. ein Verfahren, bei dem man eine Schmelze aus einem polymeren Bindemittel und einem Wirkstoff extrudiert und den extrudierten Strang zu der gewünschten Arzneiform formt, ist bekannt, siehe z. B. EP-A 240 904, EP-A 240 906, EP-A 337 256 und EP-A 358 105. Dieses Verfahren gestattet die Zubereitung schwerlöslicher Wirkstoffe in Form fester Lösungen. In den festen Lösungen liegt der Wirkstoff in amorpher Form vor und kann daher leichter resorbiert werden als der kristalline Wirkstoff. Allerdings erfolgt die Auflösung der Dosierungsform und die Freisetzung des Wirkstoffs nur an der Oberfläche der Dosierungsform. In vielen Fällen ist jedoch ein rascher Zerfall der Dosierungsform erwünscht.

Die EP-B 0078430 offenbart ein Verfahren zur Herstellung von schnell freisetzenden Arzneizubereitungen, die Dihydropyridin, Polyvinylpyrrolidon und unlösliche Trägerstoffe wie vernetztes Polyvinylpyrrolidon enthalten, wobei man den Wirkstoff und das Polyvinylpyrrolidon in einem organischen Lösungsmittel löst und die Lösung mit dem Trägerstoff granuliert. Dieses Verfahren kann allerdings nicht ohne Weiteres auf andere schwerlösliche Wirkstoffe übertragen werden, da nicht für alle Wirkstoffe ein geeignetes physiologisch verträgliches Lösungsmittel existiert und/oder eine vollständige Entfernung des Lösungsmittels nicht oder nur mit Mühe gelingt.

Die GB 2 153 676 schlägt vor, wasserunlösliche Polymere wie vernetztes Polyvinylpyrrolidon mit einem Wirkstoff zu beladen, indem man das Polymer mit dem Wirkstoff mischt und auf den Schmelzpunkt des Wirkstoffs erwärmt. Dieses Vorgehen hat den Nachteil, dass sich viele Wirkstoffe nicht unzersetzt schmelzen lassen.

Gemäß der EP-A 0 446 753 werden venetzte Polymere mit einem Wirkstoff beladen, indem man das Polymer mit einer Lösung des Wirkstoffs behandelt oder das Polymer und den Wirkstoff unter hohem Energieeintrag vermahlt. Das Verfahren hat den Nachteil, dass es nicht kontinuierlich durchgeführt werden kann.

Die DE-A 44 13 350 beschreibt Retard-Matrixpellets, die aus einem Wirkstoff, 5 bis 50 Gew.-% eines wasserunlöslichen Polymers wie Ethylcellulose, 5 bis 45 Gew.-% einer lipophilen Komponente, 3 bis 40 Gew.-% eines Gelbildners wie Hydroxypropylcellulose sowie gegebenenfalls Formulierungshilfsmitteln bestehen. Die Herstellung der Retard-Matrixpellets kann durch Schmelzextrusion erfolgen.

US 2004/012706 beschreibt ein Granulat, welches 32.6% Itraconazol, 48.9% Hydroxypropylmethylcellulose, 13% Na-Croscarmellose als Sprengmittel und 5.5% Glycerolmonostearat enthält. Die Herstellung erfolgt bei 150°C.

Der Erfindung liegt die Aufgabe zu Grunde, ein universell anwendbares Verfahren anzugeben, das die Herstellung von Dosierungsformen mit rascher Freisetzung insbesondere schwerlöslicher Wirkstoffe ohne das Erfordernis der Verwendung organischer Lösungsmittel oder des Aufschmelzens des Wirkstoffs erlaubt.

Gegenstand der vorliegenden Erfindung ist daher ein Verfahren zur Herstellung fester Dosierungsformen, bei dem man eine formbare Masse, die
a) 50 bis 99,4 Gew.-%, vorzugsweise 60 bis 80 Gew.-%, wenigstens eines vernetzten nicht-thermoplastischen Trägers,
b) 0,5 bis 30 Gew.-%, vorzugsweise 5 bis 20 Gew.-%, wenigstens eines unter thermoplastischen Polymeren, Lipiden, Zuckeralkoholen, Zuckeralkoholderivaten und Solubilisatoren ausgewählten Adjuvans und
c) 0,1 bis 49,5 Gew.-%, vorzugsweise 5 bis 25 Gew.-%, wenigstens eines Wirkstoffs

umfasst, bei einer Temperatur bei oder oberhalb des Erweichungspunkts des Adjuvans, mindestens jedoch 70 °C, vorzugsweise 100 bis 180 °C, bildet und anschließend abkühlt.

In bevorzugten Ausführungsformen umfasst die Masse
a) 50 bis 90 Gew.-%, vorzugsweise 60 bis 80 Gew.-%, wenigstens eines vernetzten nicht-thermoplastischen Trägers,
b1) 5 bis 30 Gew.-%, vorzugsweise 7 bis 15 Gew.-%, wenigstens eines thermoplastischen Polymers,
b2) 0,5 bis 20 Gew.-%, vorzugsweise 5 bis 10 Gew.-%, wenigstens eines Solubilisators,
c) 0,1 bis 45,5 Gew.-%, vorzugsweise 5 bis 25 Gew.-%, wenigstens eines Wirkstoffs.

Der vernetzte nicht-thermoplastische Träger wirkt als Sprengmittel, das einen raschen Zerfall der Dosierungsform in einer wässrigen Umgebung wie Magensaft bewirkt. Überraschenderweise gelingt die Herstellung der Dosierungsformen, die einen überwiegenden Anteil eines vernetzten nicht-thermoplastischen Trägers enthalten, in Abwesenheit von Lösungsmitteln durch ein der Schmelzextrusion ähnliches Verfahren, wenn bestimmte Adjuvantien mitverwendet werden. Unter "Adjuvans" oder "Adjuvantien" werden Hifsstoffe verstanden, die in der Dosierungsform verbleiben und nicht lediglich im Zuge der Herstellung zugesetzt und in einem späteren Bearbeitungsschritt wieder entfernt werden.

Unter Dosierungsformen sind alle Formen zu verstehen, die zur Verwendung als Arzneimittel, insbesondere zur oralen Verabreichung, Pflanzenbehandlungsmittel, Futtermittel und Nahrungsergänzungsmittel geeignet sind. Dazu gehören beispielsweise Tabletten jeglicher Form, Pellets oder Granulate.

Der vernetzte nicht-thermoplastische Träger ist ein natürliches, halbsynthetisches oder vollsynthetisches Polymer, das bis zu einem solchen Vernetzungsgrad vernetzt ist, dass es keine thermoplastischen Eigenschaften aufweist. Es ist in der Regel wasserunlöslich aber wasserquellbar. Vorzugsweise ist der nicht-thermoplastische Träger unter vernetztem Polyvinylpyrrolidon und vernetzter Natriumcarboxymethylcellulose ausgewählt. Vernetztes Polyvinylpyrrolidon ist am meisten bevorzugt. Geeignete Produkte sind z. B. in der US Pharmakopöie (USP NF) beschrieben.

Neben dem Wirkstoff und dem vernetzten nicht-thermoplastischen Träger setzt man im erfindungsgemäßen Verfahren wenigstens ein unter thermoplastischen Polymeren, Lipiden, Zuckeralkoholen, Zuckeralkoholderivaten und Solubilisatoren ausgewähltes Adjuvans ein.

Geeignete thermoplastische Polymere sind beispielsweise Polyvinylpyrrolidon (PVP), Copolymerisate von N-Vinylpyrrolidon und Vinylacetat oder Vinylpropionat, Copolymerisate von Vinylacetat und Crotonsäure, teilverseiftes Polyvinylacetat, Polyvinylalkohol, Polyhydroxyalkylacrylate, Polyhydroxyalkylmethacrylate, Polyacrylate und Polymethacrylate (Eudragit-Typen), Copolymerisate von Methylmethacrylat und Acrylsäure, Polyethylenglykole, Alkylcellulosen, insbesondere Methylcellulose und Ethylcellulose, Hydroxyalkylcellulosen, insbesondere Hydroxypropylcellulose (HPC), Hydroxyalkyl-Alkylcellulosen, insbesondere Hydroxypropylmethylcellulose (HPMC), Celluloseester wie Cellulosephthalate, insbesondere Celluloseacetatphthalat, Hydroxypropylmethylcellulosephthalat und Hydroxypropylmethylcelluloseacetatsuccinat (HPMCAS). Davon sind Homo- oder Copolymere von Vinylpyrrolidon besonders bevorzugt, z. B. Polyvinylpyrrolidon mit K-Werten nach Fikentscher von 12 bis 100, vorzugsweise 17 bis 30, oder Copolymere von 30 bis 70 Gew.-% N-Vinylpyrrolidon (VP) und 70 bis 30 Gew.-% Vinylacetat (VA), wie z. B. ein Copolymer aus 60 Gew.-% VP und 40 Gew.-% VA.

Die thermoplastischen Polymere weisen vorzugsweise eine Erweichungstemperatur von 60 bis 180 °C, insbesondere 70 bis 130 °C auf.

Geeignete Zuckeralkohole sind Sorbit, Xylit, Mannit, Maltitol; ein geeignetes Zuckeralkoholderivat ist Isomalt.

Geeignete Lipide sind Fettsäuren, wie Stearinsäure; Fettalkohole, wie Cetyl- oder Stearylalkohol; Fette, wie tierische oder pflanzliche Fette; Wachse, wie Carnaubawachs; oder Mono- und/oder Diglyceride oder Phosphatide, insbesondere Lecithin. Die Fette haben vorzugsweise einen Schmelzpunkt von wenigstens 50°C. Bevorzugt sind Triglyceride der C₁₂-, C₁₄-, C₁₆- und C₁₈-Fettsäuren.

Unter Solubilisatoren werden pharmazeutisch verträgliche, nichtionische oberflächenaktive Verbindungen verstanden. Zu den geeigneten Solubilisatoren zählen Sorbitanfettsäureester, polyalkoxylierte Fettsäureester, wie z. B polyalkoxylierte Glyceride, polyalkoxylierte Sorbitanfettsäureester oder Fettsäureester von Polyalkylenglykolen; oder polyalkoxylierte Ether von Fettalkoholen. Eine Fettsäurekette in diesen Verbindungen umfasst in der Regel 8 bis 22 Kohlenstoffatome. Die Polyalkylenoxid-Blöcke umfassen pro Molekül durchschnittlich 4 bis 50 Alkylenoxideinheiten, vorzugsweise Ethylenoxideinheiten.

Geeignete Sorbitanfettsäureester sind Sorbitanmonolaurat, Sorbitanmonopalmitat, Sorbitanmonostearat, Sorbitanmonooleat, Sorbitantristearat, Sorbitantrioleat, Sorbitanmonostearat, Sorbitanmonolaurat oder Sorbitanmonooleat.

Geeignete polyalkoxylierte Sorbitanfettsäurester sind beispielsweise Polyoxyethylen(20)sorbitanmonolaurat, Polyoxyethylen(20)sorbitanmonopalmitat, Polyoxyethylen(20)sorbitanmonostearat, Polyoxyethylen(20)sorbitanmonooleat, Polyoxyethylen(20)sorbitantristearat, Polyoxyethylen(20)sorbitantrioleat, Polyoxyethylen(4)sorbitanmonostearat, Polyoxyethylen(4)sorbitanmonolaurat oder Polyoxyethylen(4)sorbitanmonooleat.

Geeignete polyalkoxylierte Glyceride werden z. B. durch Alkoxylierung von natürlichen oder hydrierten Glyceriden bzw. durch Umesterung von natürlichen oder hydrierten Glyceriden mit Polyalkylenglykolen erhalten. Handelsübliche Beispiele sind Polyoxyethylenglycerolricinoleat-35, Polyoxyethylenglyceroltrihydroxystearat-40 (Cremophor^{®} RH40, BASF AG) sowie polyalkoxylierte Glyceride wie sie unter den Handelsnamen Gelucire^{®} und Labrafil^{®} von Gattefosse erhältlich sind, z. B. Gelucire^{®} 44/14 (Lauroyl-Macrogol-32-glyceride, hergestellt durch Umesterung von hydriertem Palmkernöl mit PEG 1500), Gelucire^{®} 50/13 (Stearoyl-Macrogol-32-glyceride, hergestellt durch Umesterung von hydriertem Palmöl mit PEG 1500) oder Labrafil M1944 CS (Oleoyl-Macrogol-6-glyceride, hergestellt durch Umesterung von Aprikosenkernöl mit PEG 300).

Ein geeigneter Fettsäureester von Polyalkylenglykolen ist z. B. PEG-660-Hydroxystearinsäure (Polyglykolester der 12-Hydroxystearinsäure (70 mol-%) mit 30 mol-% Ethylenglykol).

Geeignete polyalkoxylierte Ether von Fettalkoholen sind z. B. Macrogol-6-Cetylstearylether oder Macrogol-25-Cetylstearylether

Daneben können übliche galenische Hilfsstoffe, deren Gesamtmenge bis zu 20 Gew.-%, bezogen auf die Dosierungsform, betragen kann, mitverwendet werden. Hierzu zählen:
Streckmittel bzw. Füllstoffe, wie Lactose, Cellulose, Silikate oder Kieselsäure,
Schmiermittel, wie Magnesium- und Calciumstearat, Natriumstearylfumarat,
Farbstoffe, wie Azofarbstoffe, organische oder anorganische Pigmente oder Farbstoffe natürlicher Herkunft,
Stabilisatoren, wie Antioxidanzien, Lichtstabilisatoren, Hydroperoxid-Vernichter, Radikalfänger, Stabilisatoren gegen mikrobiellen Befall.

Unter Wirkstoffen im Sinne der Erfindung sind alle Stoffe mit einer erwünschten physiologischen Wirkung auf den menschlichen oder tierischen Körper oder Pflanzen zu verstehen. Es handelt sich insbesondere um pharmazeutische Wirkstoffe. Die Wirkstoffmenge pro Dosiseinheit kann in weiten Grenzen variieren. Sie wird in der Regel so gewählt, dass sie zur Erzielung der gewünschten Wirkung ausreicht. Auch Wirkstoff-Kombinationen können eingesetzt werden. Wirkstoffe im Sinne der Erfindung sind auch Vitamine und Mineralstoffe. Zu den Vitaminen gehören die Vitamine der A-Gruppe, der B-Gruppe, worunter neben B₁, B₂, B₆ und B₁₂ sowie Nicotinsäure und Nicotinamid auch Verbindungen mit Vitamin B-Eigenschaften verstanden werden, wie z. B. Adenin, Cholin, Pantothensäure, Biotin, Adenylsäure, Folsäure, Orotsäure, Pangamsäure, Carnitin, p-Aminobenzoesäure, myo-Inosit und Liponsäure sowie Vitamin C, Vitamine der D-Gruppe, E-Gruppe, F-Gruppe, H-Gruppe, I- und J-Gruppe, K-Gruppe und P-Gruppe. Zu Wirkstoffen im Sinne der Erfindung gehören auch Peptidtherapeutika und Proteine. Zu Pflanzenbehandlungsmitteln zählen z. B. Vinclozolin, Epoxiconazol und Quinmerac.

Das erfindungsgemäße Verfahren ist beispielsweise zur Verarbeitung folgender Wirkstoffe geeignet:

Acebutolol, Acetylcystein, Acetylsalicylsäure, Aciclovir, Albrazolam, Alfacalcidol, Allantoin, Allopurinol, Ambroxol, A-mikacin, Amilorid, Aminoessigsäure, Amiodaron, Amitriptylin, Amlodipin, Amoxicillin, Ampicillin, Ascorbinsäure, Aspartam, Astemizol, Atenolol, Beclomethason, Benserazid, Benzalkonium-Hydrochlorid, Benzocain, Benzoesäure, Betamethason, Bezafibrat, Biotin, Biperiden, Bisoprolol, Bromazepam, Bromhexin, Bromocriptin, Budesonid, Bufexamac, Buflomedil, Buspiron, Coffein, Campher, Captopril, Carbamazepin, Carbidopa, Carboplatin, Cefachlor, Cefalexin, Cefatroxil, Cefazolin, Cefixim, Cefotaxim, Ceftazidim, Ceftriaxon, Cefuroxim, Celedilin, Chloramphenicol, Chlorhexidin, Chlor-pheniramin, Chlortalidon, Cholin, Cyclosporin, Cilastatin, Cimetidin, Ciprofloxacin, Cisapride, Cisplatin, Clarithromycin, Clävulansäure, Clomibramin, Clonazepam, Clonidin, Clotrimazol, Codein, Cholestyramin, Cromoglycinsäure, Cyanocobalamin, Cyproteron, Desogestrel, Dexamethason, Dexpanthenol, Dextromethorphan, Dextropropoxiphen, Diazepam, Diclofenac, Digoxin, Dihydrocodein, Dihydroergotamin, Dihydroergotoxin, Diltiazem, Diphenhydramin, Dipyridamol, Dipyron, Disopyramid, Domperidon, Dopamin, Doxycyclin, Enalapril, Ephedrin, Epinephrin, Ergocalciferol, Ergotamin, Erythromycin, Estradiol, Ethinylestradiol, Etoposid, Eucalyptus Globulus, Famotidin, Felodipin, Fenofibrat, Fenofibrinsäure, Fenoterol, Fentanyl, Flavin-Mononucleotid, Fluconazol, Flunarizin, Fluorouracil, Fluoxetin, Flurbiprofen, Furosemid, Gallopamil, Gemfibrozil, Gentamicin, Gingko Biloba, Glibenclamid, Glipizid, Clozapin, Glycyrrhiza glabra, Griseofulvin, Guaifenesin, Haloperidol, Heparin, Hyaluronsäure, Hydrochlorothiazid, Hydrocodon, Hydrocortison, Hydromorphon, Ipratropium-Hydroxid, Ibuprofen, Imipenem, Indomethacin, Insulin, Iohexol, Iopamidol, Isosorbid-Dinitrat, Isosorbid-Mononitrat, Isotretinoin, Ketotifen, Ketoconazol, Ketoprofen, Ketorolac, Labatalon, Lactulose, Lecithin, Levocarnitin, Levodopa, Levoglutamid, Levonorgestrel, Levothyroxin, Lidocain, Lipase, Lipramin, Lisinopril, Loperamid, Lorazepam, Lovastatin, Medroxyprogesteron, Menthol, Methotrexat, Methyldopa, Methylprednisolon, Metoclopramid, Metoprolol, Miconazol, Midazolam, Minocyclin, Minoxidil, Misoprostol, Morphin, Multivitamin-Mischungen bzw. -Kombinationen und Mineralsalze, N-Methylephedrin, Naftidrofuryl, Naproxen, Neomycin, Nicardipin, Nicergolin, Nicotinamid, Nicotin, Nicotinsäure, Nifedipin, Nimodipin, Nitrazepam, Nitrendipin, Nizatidin, Norethisteron, Norfloxacin, Norgestrel, Nortriptylin, Nystatin, Ofloxacin, Omeprazol, Ondansetron, Pancreatin, Panthenol, Pantothensäure, Paracetamol, Penicillin G, Penicillin V, Phenobarbital, Phenoxifyllin, Phenoxymethylpenicillin, Phenylephrin, Phenylpropanolamin, Phenytoin, Piroxicam, Polymyxin B, Povidon-Iod, Pravastatin, Prazepam, Prazosin, Prednisolon, Prednison, Promocriptin, Propafenon, Propranolol, Proxyphyllin, Pseudoephedrin, Pyridoxin, Quinidin, Ramipril, Ranitidin, Reserpin, Retinol, Riboflavin, Rifampicin, Rutosid, Saccharin, Salbutamol, Salcatonin, Salicylsäure, Simvastatin, Somatropin, Sotalol, Spironolacton, Sucralfat, Sulbactam, Sulfamethoxazol, Sulfasalazin, Sulpirid, Tamoxifen, Tegafur, Teprenon, Terazosin, Terbutalin, Terfenadin, Tetracyclin, Theophyllin, Thiamin, Ticlopidin, Timolol, Tranexamsäure, Tretinoin, Triamcinolon-Acetonid, Triamteren, Trimethoprim, Troxerutin, Uracil, Valproinsäure, Vancomycin, Verapamil, Vitamin E, Volinsäure, Zidovudin.

Das Verfahren eignet sich besonders für Wirkstoffe mit einer Löslichkeit in Wasser bei 25 °C von weniger als 1 mg/ml. Solche Wirkstoffe werden gemäß USP XXII, S. 8, auch als kaum löslich oder praktisch unlöslich bezeichnet.

Zur Herstellung der festen Dosierungsformen wird bei einer erhöhten Temperatur, d. h. einer Temperatur bei oder oberhalb des Erweichungspunkts des Adjuvans, mindestens jedoch 70 °C, eine formbare kohäsive Masse aus den Komponenten hergestellt, die anschließend, gegebenenfalls nach einem Formgebungsschritt, abgekühlt wird. Vorzugsweise beträgt die Zeitspanne, über die die Komponenten der erhöhten Temperatur ausgesetzt sind, für jede der Komponenten weniger als 5 Minuten, insbesondere weniger als 3 Minuten.

Das Vermischen der Komponenten und die Bildung der formbaren Masse können auf unterschiedliche Weise erfolgen. Das Vermischen kann vor, während und/oder nach dem Erwärmen einzelner oder aller Komponenten der Masse erfolgen, wobei es jedoch nicht zweckmäßig ist, den vernetzten nicht-thermoplastischen Träger in Abwesenheit der thermoplastischen Komponenten der Masse zu erwärmen. Beispielsweise können die Komponenten zuerst vermischt und dann zur Bildung der formbaren Masse erwärmt werden. Sie können aber auch gleichzeitig vermischt und erwärmt werden. Häufig erfolgt noch eine Homogenisierung der formbaren Masse, um eine hochdisperse Verteilung des Wirkstoffes zu erhalten. Bei empfindlichen Wirkstoffen schmilzt man vorzugweise zunächst das bzw. die Adjuvantien in Gegenwart des nicht-thermoplastischen Trägers auf und mischt dann den Wirkstoff zu.

Das Erwärmen erfolgt in einer für diesen Zweck üblichen Vorrichtung. Besonders geeignet sind beheizbare Extruder oder Kneter, wie Misch-Knetreaktoren (z. B. ORP, CRP, AP, DTB der Firma List oder Reactotherm der Firma Krauss-Maffei oder Ko-Kneter der Fa. Buss), Doppelmuldenkneter (Trogmischer) und Stempelkneter (Innenmischer) oder Rotor/Stator-Systeme (z. B. Dispax der Firma IKA). Die Verweildauer der Masse im Extruder beträgt vorzugsweise weniger als 5 Minuten, insbesondere weniger als 3 Minuten.

Als Extruder kann man Einschneckenmaschinen, kämmende Schneckenmaschinen oder auch Mehrwellextruder, insbesondere Zweischnecken-Extruder, gleichsinnig oder gegensinnig drehend und gegebenenfalls mit Knetscheiben ausgerüstet, einsetzen. Besonders bevorzugt sind Doppelschneckenextruder der ZSK-Baureihe von Werner u. Pfleiderer.

Das Beschicken des Extruders bzw. Kneters erfolgt je nach deren Konzeption kontinuierlich oder diskontinuierlich in üblicher Weise. Pulverförmige Komponenten können im freien Zulauf, z. B. über eine Differentialdosierwaage eingeführt werden. Plastische Massen können direkt aus einem Extruder eingespeist oder über eine Zahnradpumpe, die insbesondere bei hohen Viskositäten und hohen Drücken von Vorteil ist, zugespeist werden. Flüssige Medien können über ein geeignetes Pumpenaggregat zudosiert werden.

Die erhaltene Masse ist teigig bis pastös. Sie wird in der Regel einer Formgebung unterzogen. Dabei kann eine Vielzahl von Formen, je nach Werkzeug und Art der Formung, erzeugt werden. Beispielsweise lässt sich bei Verwendung eines Extruders der extrudierte Strang zwischen einem Band und einer Walze, zwischen zwei Bändern oder zwischen zwei Walzen, wie in der EP-A-358 105 beschrieben, oder durch Kalandrierung in einem Kalander mit zwei Formwalzen, siehe beispielsweise EP-A-240 904, formen.

Durch Extrusion und Heiß- oder Kaltabschlag des Stranges können beispielsweise kleinteilige Granulate erhalten werden. Die erkalteten Massen können anschließend auch zu Pulver gemahlen und dann in üblicher Weise zu Tabletten verpresst werden. Hierbei können Tablettierhilfsmittel wie kolloidale Kieselsäure, Calciumhydrogenphosphat, Lactose, mikrokristalline Cellulose, Stärke oder Magnesiumstearat mitverwendet werden.

Die Erfindung wird durch die folgenden Beispiele näher veranschaulicht.

### Beispiele

### Beispiel 1

Ein Gemisch von 20,83 Gew.-% Wirkstoff (Lopinavir), 68,17 Gew.-% vernetztem Polyvinylpyrrolidon (Kollidon CL), 7,00 Gew.-% Polyoxyethylenglyceroltrihydroxystearat-40 (Cremophor^{®} RH-40) und 1,00 Gew.- Aerosil 200 wurde in einem Doppelschnecken-Extruder (18 mm Schneckendurchmesser) bei einer Materialtemperatur von 120 °C verarbeitet. Das Cremophor^{®} RH-40 war zuvor bei Raumtemperatur mit dem pulverförmigen Kollidon CL unter Rühren bzw. Kneten zu einem rieselfähigen Granulat vermischt worden, dem dann der Wirkstoff und das Aerosil 200 beigemischt wurden. Über eine Dosierwaage wurden 1,5 kg/h dieses Gemisches in den Extruder dosiert. Aus dem Extruder-Kopf trat eine heiße formbare Masse in Gestalt eines weißen Strang-Extrudats aus, das nach dem Abkühlen erhärtete. Die erkalteten Extrudat-Stränge (mit einer Dicke von etwa 10 mm) zerfielen in Wasser in wenigen Minuten.

### Beispiel 2

Stücke des in Beispiel 1 erhaltenen Extrudats wurden in einer Labormühle (Fa. Retsch) gemahlen und nach Zugabe von 12 Gew.-% Calciumhydrogenphosphat und 1 Gew.-% Aerosil 200 (kolloidale Kieselsäure) auf einer Exzenterpresse (Fette E 1) zu Oblong-Tabletten verpresst. Die Tabletten zeigten im Zerfallstest (gemäß DAB) in 0,1 M Salzsäure bei 37 °C eine Zerfallszeit von wenigen Minuten.

### Beispiel 3 (Vergleichsbeispiel)

Beispiel 1 wurde wiederholt, wobei jedoch ein Copolymer aus 60 Gew.-% N-Vinylpyrrolidon und 40 Gew.-% Vinylacetat (Kollidon VA-64) anstelle von Kollidon CL verwendet wurde. Aus dem Extruderkopf trat ein durchscheinender Schmelzestrang aus, der nach dem Ankülen eine harte spröde Masse bildete. Die Extrudat-Stränge lösten sich in Wasser erst nach mehreren Stunden auf.

### Beispiel 4 (Vergleichsbeispiel)

Stücke des in Beispiel 3 erhaltenen Extrudats wurden analog zu Beispiel 2 gemahlen und mit den angegebenen Hilfstoffen zu Oblong-Tabletten verpresst. Die Zerfallszeit der Tabletten im Zerfallstest (gemäß DAB) betrug mehr als 3 Stunden.

### Beispiel 5

Analog zu Beispiel 1 wurde ein Gemisch aus 20,83 Gew.-% Wirkstoff (Lopinavir), 61,17 Gew.-% vernetztem Polyvinylpyrrolidon (Kollidon CL), 10,00 Gew.-% N-Vinylpyrrolidon/Vinylacetat-Copolymer 60/40 (Kollidon VA-64), 7,00 Gew.-% Cremophor RH-40 und 1,00 Gew.- Aerosil 200 verarbeitet. Aus dem Extruder-Kopf trat eine heiße formbare Masse in Gestalt eines weißen Strang-Extrudats aus, das nach dem Abkühlen erhärtete. Die erkalteten Extrudat-Stränge zerfielen in Wasser in wenigen Minuten.

### Beispiel 6

Analog zu Beispiel 1 wurde ein Gemisch aus 20,83 Gew.-% Wirkstoff (Lopinavir), 51,17 Gew.-% vernetztem Polyvinylpyrrolidon (Kollidon CL), 20,00 Gew.-% N-Vinylpyrrolidon/ Vinylacetat-Copolymer 60/40 (Kollidon VA-64), 7,00 Gew.-% Cremophor RH-40 und 1,00 Gew.- Aerosil 200 verarbeitet. Aus dem Extruder-Kopf trat eine heiße formbare Masse in Gestalt eines weiß-gelblichen Strang-Extrudats aus, das nach dem Abkühlen erhärtete. Die erkalteten Extrudat-Stränge zerfielen in Wasser in wenigen Minuten.

### Beispiel 7

Analog zu Beispiel 1 wurde ein Gemisch aus 20,83 Gew.-% Wirkstoff (Lopinavir), 61,17 Gew.-% vernetztem Polyvinylpyrrolidon (Kollidon CL), 10,00 Gew.-% N-Vinylpyrrolidon/ Vinylacetat-Copolymer 60/40 (Kollidon VA-64), 7,00 Gew.-% Sorbitanmonopalmitat (Span 40) und 1,00 Gew.- Aerosil 200 verarbeitet. Aus dem Extruder-Kopf trat eine heiße formbare Masse in Gestalt eines weiß-gelblichen Strang-Extrudats aus, das nach dem Abkühlen erhärtete. Die erkalteten Extrudat-Stränge zerfielen in Wasser in wenigen Minuten.

## Patentansprüche

1. Verfahren zur Herstellung fester Dosierungsformen, bei dem man eine formbare Masse, die
a) 50 bis 99,4 Gew.-% wenigstens eines vernetzten nicht-thermoplastischen Trägers,
b) 0,5 bis 30 Gew.-% wenigstens eines unter thermoplastischen Polymeren, Lipiden, Zuckeralkoholen, Zuckeralkoholderivaten und Solubilisatoren ausgewählten Adjuvans und
c) 0,1 bis 49,5 Gew.-% wenigstens eines Wirkstoffs
umfasst, bei einer Temperatur bei oder oberhalb des Erweichungspunkts des Adjuvans, mindestens jedoch 70 °C, bildet und anschließend abkühlt.

2. Verfahren nach Anspruch 1, wobei die Masse umfasst
a) 50 bis 90 Gew.-% wenigstens eines vernetzten nicht-thermoplastischen Trägers,
b1) 5 bis 30 Gew.-% wenigstens eines thermoplastischen Polymers,
b2) 0,5 bis 20 Gew.-% wenigstens eines Solubilisators,
c) 0,1 bis 45,5 Gew.-% wenigstens eines Wirkstoffs.

3. Verfahren nach Anspruch 1 oder 2, wobei der vernetzte nicht-thermoplastische Träger ausgewählt ist unter vernetztem Polyvinylpyrrolidon und vernetzter Natriumcarboxymethylcellulose.

4. Verfahren nach einem der vorhergehenden Ansprüche, wobei das thermoplastisches Polymer ein Homo- oder Copolymer von Vinylpyrrolidon ist.

5. Verfahren nach einem der vorhergehenden Ansprüche, wobei der Zuckeralkohol ausgewählt ist unter Sorbit, Xylit, Mannit, Maltitol und das Zuckeralkoholderivat Isomalt ist.

6. Verfahren nach einem der vorhergehenden Ansprüche, wobei das Lipid ausgewählt ist unter Fettsäuren, Fettalkoholen, Fetten, Wachsen, Mono- und Diglyceriden und Phosphatiden.

7. Verfahren nach einem der vorhergehenden Ansprüche, wobei der Solubilisator ausgewählt ist unter Sorbitanfettsäureestern, polyalkoxylierten Fettsäureestern und polyalkoxylierten Ethern von Fettalkoholen.

8. Verfahren nach einem der vorhergehenden Ansprüche, wobei der Wirkstoff eine Löslichkeit in Wasser bei 25 °C von weniger als 1 mg/ml aufweist.

9. Verfahren nach einem der vorhergehenden Ansprüche, wobei man die abgekühlte Masse zerkleinert und zu der Dosierungsform verpresst.

## Claims

1. A process for producing solid dosage forms, in which a moldable composition which comprises
a) 50 to 99.4% by weight of at least one crosslinked nonthermoplastic carrier,
b) 0.5 to 30% by weight of at least one adjuvant selected from thermoplastic polymers, lipids, sugar alcohols, sugar alcohol derivatives and solubilizers and
c) 0.1 to 49.5% by weight of at least one active ingredient,
is formed at a temperature at or above the softening point of the adjuvant, but at least 70°C, and subsequently cooled.

2. The process according to claim 1, where the composition comprises
a) 50 to 90% by weight of at least one crosslinked nonthermoplastic carrier,
b1) 5 to 30% by weight of at least one thermoplastic polymer,
b2) 0.5 to 20% by weight of at least one solubilizer,
c) 0.1 to 45.5% by weight of at least one active ingredient.

3. The process according to claim 1 or 2, where the crosslinked nonthermoplastic carrier is selected from crosslinked polyvinylpyrrolidone and crosslinked sodium carboxymethylcellulose.

4. The process according to any of the preceding claims, where the thermoplastic polymer is a homo- or copolymer of vinylpyrrolidone.

5. The process according to any of the preceding claims, where the sugar alcohol is selected from sorbitol, xylitol, mannitol, maltitol and the sugar alcohol derivative isomalt.

6. The process according to any of the preceding claims, where the lipid is selected from fatty acids, fatty alcohols, fats, waxes, mono- and diglycerides and phosphatides.

7. The process according to any of the preceding claims, where the solubilizer is selected from sorbitan fatty acid esters, polyalkoxylated fatty acid esters and polyalkoxylated ethers of fatty alcohols.

8. The process according to any of the preceding claims, where the active ingredient has a solubility in water at 25°C of less than 1 mg/ml.

9. The process according to any of the preceding claims, where the cooled composition is comminuted and compressed to the dosage form.

## Revendications

1. Procédé pour produire des formes galéniques solides, dans lequel on forme une masse pouvant être mise en forme qui comprend
a) 50 à 99,4 % en masse d'au moins un support non thermoplastique réticulé,
b) 0,5 à 30 % en masse d'au moins un adjuvant choisi parmi les polymères thermoplastiques, les lipides, les alcools de sucre, les dérivés d'alcool de sucre et les solubilisants et
c) 0,1 à 49,5 % en masse d'au moins un principe actif
à une température à ou au-delà du point de ramollissement de l'adjuvant, mais d'au moins 70°C, après quoi on la refroidit.

2. Procédé selon la revendication 1 où la masse comprend
a) 50 à 90 % en masse d'au moins un support non thermoplastique réticulé,
b1) 5 à 30 % en masse d'au moins un polymère thermoplastique,
b2) 0,5 à 20 % en masse d'au moins un solubilisant,
c) 0,1 à 45,5 % en masse d'au moins un principe actif.

3. Procédé selon la revendication 1 ou 2 où le support non thermoplastique réticulé est choisi parmi la polyvinylpyrrolidone réticulée et la carboxyméthylcellulose sodique réticulée.

4. Procédé selon l'une des revendications précédentes où le polymère thermoplastique est un homo- ou copolymère de vinylpyrrolidone.

5. Procédé selon l'une des revendications précédentes où l'alcool de sucre est choisi parmi le sorbitol, le xylitol, le mannitol, le maltitol et le dérivé d'alcool de sucre isomalt.

6. Procédé selon l'une des revendications précédentes où le lipide est choisi parmi les acides gras, les alcools gras, les graisses, les cires, les mono- et diglycérides et les phosphatides.

7. Procédé selon l'une des revendications précédentes où le solubilisant est choisi parmi les esters de sorbitan et d'acide gras, les esters d'acide gras polyalcoxylés et les éthers d'alcools gras polyalcoxylés.

8. Procédé selon l'une des revendications précédentes où le principe actif présente une solubilité dans l'eau à 25°C inférieure à 1 mg/ml.

9. Procédé selon l'une des revendications précédentes où l'on fractionne la masse refroidie et on la compresse en la forme galénique.
